**Europäisches Patentamt**

**European Patent Office**

**Office européen des brevets**

(19)

(11) Veröffentlichungsnummer: **0 187 206**

**A1**

(12)

# EUROPÄISCHE PATENTANMELDUNG

(21) Anmeldenummer: **85112964.3**

(22) Anmeldetag: **12.10.85**

(51) Int. Cl.⁴: **C 07 C 19/045**
    **C 07 C 17/02**

(30) Priorität: **15.12.84 DE 3445896**

(43) Veröffentlichungstag der Anmeldung:
    **16.07.86 Patentblatt 86/29**

(84) Benannte Vertragsstaaten:
    **AT BE CH DE FR GB IT LI LU NL SE**

(71) Anmelder: **DYNAMIT NOBEL AKTIENGESELLSCHAFT**
    **Postfach 1261**
    **D-5210 Troisdorf, Bez. Köln(DE)**

(72) Erfinder: **Piotrowski, Bernhard, Dr.**
    **Alte Lohmarer Strasse 84**
    **D-5204 Lohmar 1(DE)**

(72) Erfinder: **Schildhauer, Roland**
    **Adenauerplatz 3**
    **D-5216 Niederkassel 5(DE)**

(72) Erfinder: **Deselaers, Kurt, Dr.**
    **Röntgenstrasse 2**
    **D-5210 Troisdorf(DE)**

(72) Erfinder: **Merkel, Wolfgang, Dr.**
    **Auf dem Grend 23**
    **D-5210 Troisdorf(DE)**

(54) **Verfahren zur Herstellung von 1,2-Dichlorethan.**

(57) Es wird ein Verfahren zur Herstellung von 1.2-Dichlorethan in einem Reaktor durch Umsetzung von gasförmigem Ethylen mit in einem heißen, unter erhöhtem Druck stehenden, katalysatorhaltigen, flüssigen Kreislaufstrom, welcher aus chlorierten Kohlenwasserstoffen besteht, gelöstem Chlor beschrieben. Dabei wird das gesamte Chlor ausserhalb des Reaktors bei einer Temperatur oberhalb 90°C, einem Druck von mehr als 4 bar und mit einer mittleren Verweilzeit von weniger als 120 Sekunden absorbiert. Die Reaktion findet an der Phasengrenzfläche einer aus gasförmigem Ethylen und dem chlorhaltigen, flüssigen Kreislaufstrom erzeugten Dispersion bei einer Energiedissipationsdichte von 0,05 bis 1000 kW/m³, einer Temperatur von 90 bis 200°C und einem Druck von 7 bis 20 bar statt. Als Katalysator wird vorzugsweise Eisen-III-chlorid verwendet. Als Inhibitor zur Verhinderung der Nebenproduktbildung wird vorzugsweise Sauerstoff eingesetzt. Die Trennung der nicht umgesetzten Mengen an Ethylen und Chlor sowie weiterer gasförmiger Bestandteile vom produkthaltigen Kreislaufstrom erfolgt vorzugsweise in einem Entspannungsgefäß.

./...

Dynamit Nobel Aktiengesellschaft, Troisdorf

1

Troisdorf, den 16.11.1984
Me/P. - OZ: 84065    (4358)

DYNAMIT NOBEL AKTIENGESELLSCHAFT

5210 Troisdorf

## Verfahren zur Herstellung von 1.2-Dichlorethan

Die Erfindung betrifft ein Verfahren der im Oberbegriff von Patentanspruch 1 angegebenen Art.

Die Chlorierung von Ethylen zu 1.2-Dichlorethan (EDC) ist eine exotherme Reaktion,bei der Reaktionswärme freigesetzt wird, die zur Dampferzeugung von etwa 1 t Dampf/t EDC geeignet ist (EP-OS 0075 742).

Zur Rückgewinnung dieser Reaktionswärme bei möglichst hoher EDC-Ausbeute, hohem Ethylen-Umsatz und hoher Raumzeitausbeute werden in den bekannten Verfahren verschiedene Wege beschritten  (DE-OS 29 35 884, DE-OS 24 27 045). So wird die Reaktionswärme entweder im Reaktor durch innerhalb des Reaktors installierte Kühlsysteme und durch Verdampfungskühlung des Reaktorinhaltes oder außerhalb des Reaktors durch Produktkühlung und Verdampfung abgeführt.

Weitere Unterschiede liegen in der Art der Einspeisung der Reaktanden Chlor und Ethylen in das Reaktionssystem,

- 2 -

- 2 -

der Ausgestaltung des Reaktionssystems und in der Art und Weise der Anwendung von Katalysatoren zur Förderung der gewünschten Additionsreaktion zu EDC und von Inhibitoren zur Verminderung von unerwünschten Substitutionsreaktionen, wie z.B. der Bildung von 1.1.2-Trichlorethan und anderer höhersiedender Chlorkohlenwasserstoffe.

Der Forderung nach einem vertretbaren Aufwand für die Rückgewinnung der Reaktionswärme kommen insbesondere solche Verfahren nahe, bei denen die Reaktion oberhalb $100^{o}C$ unter einem Druck von mehr als 3 bar und unter Einbindung des Reaktionssystems in einen Produktkreislauf durchgeführt wird. Dabei erfolgt die katalysatorfreie Produktausschleusung aus dem katalysatorhaltigen Produktkreislauf in der Regel durch Entspannungsverdampfung.

Wie allgemein bekannt ist, wird der Wirkungsgrad bei der Energierückgewinnung und insbesondere die Möglichkeit, technisch brauchbaren Dampf zu erzeugen, mit zunehmendem Temperaturniveau des gesamten Produktkreislaufes größer. Demgegenüber ist auch allgemein bekannt, daß mit zunehmender Temperatur im Reaktor und im Produktkreislauf die Nebenproduktbildung, d.h. Ausbeuteverluste an EDC, z.B. durch Substitutionsreaktionen und dehydrochlorierende Spaltung von EDC, gefördert wird.
Neben diesem reaktionstechnischen Aspekt mit den entsprechenden Einflußfaktoren, wie z.B. Katalysatoren, Inhibitoren, wird die technisch maximal erreichbare Reaktionstemperatur bei der Ethylenchlorierung und damit auch der Reaktionsdruck von der Höhe des Chlorvordruckes bestimmt. Hierbei wird das Chlor üblicherweise direkt aus einer Chlor-Alkali-Elektrolyse kommend als Zellenchlor nach der Kompression auf etwa 3 bar in den Reaktor eingeleitet.

- 3 -

Die Maßnahme, bei der Herstellung von EDC den Chlor-vordruck auf Werte $\geq$ 3 bar zu erhöhen, um dementsprechend Reaktionstemperaturen $\geq$ 100°C zu erreichen, basiert auf der direkten Erhöhung des Chlorvordrucks durch eine technisch aufwendige und kostenintensive weitere Chlorkompression oder auf einer indirekten Erhöhung des Chlorvordrucks durch Absorption von Chlor in EDC und der in der Technik allgemein angewandten Druckerhöhung bei einer Flüssigkeit mittels einer Pumpe.

Diese technisch einfache und kostengünstige Methode der Chlorabsorption im kalten Produktstrom mit anschließender Druckerhöhung zeigt nach den bisher bekannten Ergebnissen beim EDC keine befriedigende Lösung für eine optimale Energierückgewinnung der Reaktionswärme, wie z.B. durch Dampferzeugung, und für das Problem der Nebenproduktbildung bei diesem Verfahren.

Die wesentlichen Nachteile der bisher bekannten Anwendung der Chlorabsorptions-Druckerhöhungs-Methode sind folgende:

- Die erforderliche Abkühlung des Produktkreislaufes auf Temperaturen kleiner als 40°C zur Durchführung der hierbei gewünschten Chlorabsorption und die anschließende Erwärmung des chlorhaltigen EDC erfordert einen beträchtlichen Energieaufwand.

- Durch die relativ hohe Chlorkonzentration im EDC von größer als 8 Gew.-% bei der Chlorabsorption kann auch bei Verwendung von Inhibitoren, wie z.B. Sauerstoff, eine unerwünschte Nebenproduktbildung, insbesondere durch Substitutionsreaktionen, wegen des Erfordernisses des Hochheizens dieser Lösung bis nahe der Reaktionstemperatur und der damit verbundenen langen Verweilzeit zwischen der Chlorabsorption und dem Reaktionsbeginn im Reaktor nicht vermieden werden.

Diese bisher nicht befriedigende Anwendung der Chlorabsorptions-Druckerhöhungs-Methode hat einen weiteren nachteiligen Effekt. So ist beim Erhitzen dieser chlorhaltigen EDC-Lösung auf nahezu Reaktionstemperatur ein Ausgasen des Chlors aus dem EDC unvermeidlich. Es bildet sich eine Zweiphasen-Strömung mit hohen Chlorkonzentrationen an der Phasengrenzfläche und hoher Temperatur. Dadurch werden die vorgenannten Nebenreaktionen begünstigt.

Die bisher bekannten Maßnahmen zur Verminderung von Nebenreaktionen bei einer Reaktionstemperatur oberhalb 100°C beruhen neben der vorteilhaften Verwendung geeigneter Katalysatoren und Inhibitoren im Reaktionssystem maßgeblich auf fluiddynamischen Einflußfaktoren. So wurde beispielsweise versucht, durch eine sehr feine und möglichst homogene Verteilung der Reaktanden Inhomogenitäten der Konzentrationen und Temperaturen, wie sie z.B. beim Zusammentreffen von Chlor und Ethylenblasen eintreten können, zu vermeiden. Darunter sind auch Bestrebungen zu verstehen, das Zusammentreffen der gasförmigen Reaktanden Chlor und Ethylen soweit wie möglich auszuschließen, indem bevorzugt Chlor in EDC gelöst wird und diese Lösung mit dem gasförmigen, möglichst fein verteilten Ethylen zur Reaktion gebracht wird.

Diese Reaktionsführung über die Phasengrenze wird beispielsweise in der EP-OS 0075 742 und der DE-PS 33 40 624 aufgegriffen. Zur Durchführung dieser Absicht wird in beiden Fällen ein Reaktor gewählt, der die typischen Merkmale eines Schlaufenreaktors, wie z.B. Produkteinspeisung im unteren Teil des Reaktors mit dem hierfür charakteristischen Strömungsverlauf - im zentralen Innenrohr nach oben und im äußeren Bereich nach unten -, und eine hinreichend große Umlaufgeschwindigkeit der Strömung von beispielsweise 30 bis 200 $\frac{kg}{s\,m^2}$ oder größer als 0,1 m/s

in der Mischzone des Innenrohres aufweist.

Die hier genannten Verfahrensbeispiele erreichen die beabsichtigte Reaktionsführung an der Phasengrenzfläche nur teilweise. So erfolgt zwar bei diesen Ausführungsformen des Reaktors die feine Verteilung der Ethylenblasen vornehmlich in einer entsprechenden Düse am Boden des Reaktors und im Innenrohr des Schlaufenreaktors, jedoch kann das Entweichen der Ethylenblasen aus der reaktiven Phasengrenzfläche der flüssigen, katalysator- und chlorhaltigen Reaktionsphase in den darüber befindlichen Gasraum, bestehend aus dampfförmigem EDC, nicht umgesetzten Reaktanden, wie Chlor und insbesondere auch Ethylen, sowie anderen inerten gasförmigen Bestandteilen trotz der angegebenen Geschwindigkeiten der umlaufenden Strömung nicht vollständig vermieden werden. Insbesondere bei Verwendung eines Siedereaktors werden infolge der auftretenden Dampfblasenbildung im oberen Bereich der flüssigen Reaktionsphase die hier befindlichen Ethylenblasen im verstärkten Maße mitgerissen und somit der gewünschten Reaktion an der Phasengrenzfläche entzogen.

Der Erfindung lag die Aufgabe zugrunde, die bekannten Verfahrensweisen dahingehend zu verbessern, daß eine Chlorabsorption bei hoher Temperatur unter geringster Nebenproduktbildung durchgeführt werden kann, ohne die vorher beschriebene nachteilige Abkühlung und anschliessende Erhitzung des Produktstromes durchführen zu müssen Des weiteren bestand die Aufgabe, für die vorteilhafte Reaktion an der Phasengrenzfläche durch Vermeidung einer Phasentrennung in eine disperse Gas-Flüssig-Phase und eine darüber befindliche gasförmige Phase die geeigneten Bedingungen zu finden.

Diese Aufgaben werden gemäß den kennzeichnenden Merkmalen des Anspruchs 1 gelöst. In den Unteransprüchen sind vor-

- 6 -

teilhafte Ausgestaltungen der Erfindung dargestellt.

Es wurde gefunden, daß bei der Chlorabsorption und dem anschließenden Transport bzw. der Lagerung einer chlorhaltigen EDC-Lösung bei höherer Temperatur bestimmte Bedingungen eingehalten werden müssen, um die unerwünschte Nebenproduktbildung gering zu halten. So ist es z.B. vorteilhaft, bei der Chlorabsorption und beim Transport der chlorhaltigen EDC-Lösung bei $100^{\circ}$C im Kreislaufstrom zwischen der Einleitstelle des gasförmigen Chlors und dem Reaktor eine Chlorkonzentration von weniger als 3 Gew.-% einzuhalten. Des weiteren sollte im Kreislaufstrom eine mittlere Verweilzeit von der Zugabe des Chlors zwecks Absorption bis zum Reaktionsbeginn mit Ethylen von weniger als 120 Sekunden eingehalten werden. Darüberhinaus ist die zusätzliche Verwendung von Inhibitoren zur Verhinderung der Nebenproduktbildung vorteilhaft. So inhibiert z. B. die Zugabe von Sauerstoff Substitutionsreaktionen,d.h. die Bildung von 1.1.2-Trichlorethan und anderer höhersiedender Chlorkohlenwasserstoffe. Es wurde gefunden, daß vorteilhaft als Inhibitor Sauerstoff oder ein Sauerstoff enthaltendes Gas in einem Anteil von 0,05 bis 0,3 Gew.-%, berechnet als $O_2$ und bezogen auf die zur Reaktion eingesetzte Chlormenge, verwendet wird.

Auch andere, bekannte Inhibitoren, wie z.B. Kresole, können beim erfindungsgemäßen Verfahren allein oder zusammen mit Sauerstoff verwendet werden.

Beim erfindungsgemäßen Verfahren können für die Reaktion zwischen Chlor und Ethylen zu 1.2-Dichlorethan bekannte Katalysatoren ( s. DE-OS 29 35 884 und EP-OS 0111 203) eingesetzt werden. Insbesondere die Verwendung von Eisen-III-chlorid oder einer Eisen-III-chlorid enthaltenden Verbindung als

- 7 -

Katalysator hat sich als vorteilhaft erwiesen. Der Katalysator wird bei der Füllung des Reaktionssystems mit EDC über einen Lösebehälter zugegeben. Vorzugsweise ist er in einer Menge von 30 bis 3000 Gew.-ppm, berechnet als $FeCl_3$, im Kreislaufstrom enthalten.

Um die beim erfindungsgemäßen Verfahren geforderte begrenzte Verweildauer des Chlors bei dessen Absorption in EDC erreichen zu können, sind für diese Verfahrensstufe Stoffaustauschapparate mit einer spezifischen Austauschfläche von mindestens $10^3 \frac{m^2}{m^3}$ , wie z.B. Strahlwäscher, besonders geeignet.

Ein weiterer Beitrag zur Lösung der der Erfindung zugrundeliegenden Aufgabe besteht in der Erkenntnis, daß für die Ausbeute sowie den Temperatur- und den Konzentrationsverlauf der Reaktion, d.h. auch für die Selektivität bei relativ hohen Temperaturen, eine geeignete Energiedissipationsdichte im Reaktor herrschen muß, nämlich von 0,05 bis 1000 $kW/m^3$ an der Phasengrenzfläche der aus gasförmigem Ethylen und chlorhaltigem, flüssigem Kreislaufstrom erzeugten Dispersion. Die Energiedissipationsdichte der genannten Größe ist erforderlich, da die spezifische Oberfläche der Ethylenblasen beim erfindungsgemäßen Verfahren homogen verteilt wird. Die erfindungsgemäß bemessene Energiedissipationsdichte wirkt der Koaleszenzneigung der bei Einleitung des Ethylens in den Reaktor feinen Ethylenblasen zu großen Blasen entgegen und trägt damit dazu bei, daß eine hohe spezifische Oberfläche an Ethylen für die Reaktion zur Verfügung steht. Des weiteren wird durch die erfindungsgemäße Energiedissipationsdichte eine durch Koaleszenz der Ethylenblasen hervorgerufene Phasentrennung im Reaktor in eine gasförmige Phase im oberen Teil und eine heterogene Gas-Flüssig-Phase im unteren Teil des Reaktors vermieden.

Die erfindungsgemäße Energiedissipationsdichte wird bereits bei der Dispergierung des gasförmigen Ethylens im flüssigen, chlorhaltigen Kreislaufstrom in einer hierfür geeigneten Vorrichtung, wie z.B. einem Static-Mischer oder einer Strahl-Düse, erzeugt und beibehalten.

Es hat sich auch als vorteilhaft erwiesen, die gegebenenfalls hinzugefügten Inhibitoren in die Mischzone des Reaktors einzuleiten und dort mit einer Energiedissipationsdichte von 10 bis 1000 kW/m$^3$ zu verteilen. Überraschenderweise wurde darüberhinaus gefunden, daß die vorher beschriebene Koaleszenzneigung reduziert werden kann, d.h. die Ethylenblasen bei vergleichbaren fluiddynamischen Bedingungen länger stabil bleiben, wenn die Konzentration des gelösten Chlors im Kreislaufstrom am Austritt aus dem Reaktor mindestens 100 Gew.-ppm beträgt.

Bei der obenerwähnten Dispergierung des Ethylens in z. B. einem statischen Mischer dient dieser gleichzeitig als Reaktionszone, d.h. ein wesentlicher Anteil der Reaktanden Ethylen und Chlor reagiert bereits in der Mischvorrichtung zu 1.2-Dichlorethan.

Anschließend wird die hochdisperse Reaktionsphase derart in ein im Reaktor befindliches zentrales Impulsaustauschrohr geleitet, daß sich in der Rohrströmung und im äußeren Rand des Rohres eine Energiedissipationsdichte von 0,05 bis 1000 kW/m$^3$ einstellt. Diese so in Bewegung gebrachte Gas-Flüssig-Dispersion verläßt über eine Umlenkung im unteren Teil den Reaktor, ohne daß eine Phasentrennung im Reaktor auftreten kann. Dabei hat sich bei einer mittleren Verweilzeit von weniger als 60 Minuten der weitaus größte Teil des Ethylens mit dem Chlor zu EDC umgesetzt. Der noch nicht umgesetzte Teil des Ethylens in den verbleibenden Gasblasen kann in einem

- 9 -

dem Reaktor nachgeschalteten Entspannungsgefäß zusammen mit dem gebildeten gasförmigen EDC von dem katalysatorhaltigen Kreislaufstrom getrennt werden. Die Rückgewinnung des gasförmigen EDC von den anderen gasförmigen Bestandteilen erfolgt in Kondensatoren durch Kühlung mit Wasser und/oder Sole. Während das katalysatorfreie EDC gegebenenfalls weiteren Aufarbeitungsstufen zugeführt wird, werden die noch verbleibenden gasförmigen Bestandteile gegebenenfalls einer Nachreaktionsstufe oder direkt einer Abgasaufarbeitung zugeführt.

Neben der Energierückgewinnung durch Kondensation des gasförmigen EDC aus dem Entspannungsgefäß wird beim erfindungsgemäßen Verfahren der größte Teil der freiwerdenden Reaktionswärme direkt aus dem flüssigen, katalysatorhaltigen EDC-Kreislaufstrom durch geeignete Wärmeaustauscher zur Dampferzeugung und zur Sumpfbeheizung von Destillationskolonnen entnommen.

Eine andere erfindungsgemäße, besonders vorteilhafte Ausführungsform für die Energierückgewinnung aus dem EDC-Kreislauf basiert auf der zusätzlichen Kompression von gasförmigem EDC, wobei ein Teil bis zu einem Vielfachen des produzierten EDC aus dem Kreislaufstrom 18 in einem weiteren Entspannungsgefäß 12a verdampft, anschließend durch Verdichtung auf eine höhere Temperatur gebracht und, z.B. durch Erzeugung von Wasserdampf, gekühlt und kondensiert wird. Hierbei wurde überraschenderweise gefunden, daß eine Wärmebehandlung von EDC bei 165$^{o}$C und entsprechendem Dampfdruck in einem Zeitraum von 30 Minuten nicht zur unerwünschten Nebenproduktbildung führte.

Der flüssige, katalysatorhaltige EDC-Kreislaufstrom wird zur Chlorabsorption zurückgeleitet.

- 10 -

Die Erfindung wird nachfolgend anhand der Zeichnungen und der Beispiele näher erläutert. Es zeigen

Fig. 1    ein schematisches Verfahrensfließbild,

Fig. 2    ein schematisches Verfahrensfließbild gemäß Fig. 1, welches durch die Darstellung der zusätzlichen Energierückgewinnung ergänzt ist.

Chlorgas 1, z.B. aus einer Chlor-Alkali-Elektrolyse, mit einem Inertgas-Anteil von bis zu 10 Vol.-%, welcher im wesentlichen aus Stickstoff, Wasserstoff und Kohlendioxid besteht, mit einem Druck von etwa 3,2 bar wird mit Luft 2a versetzt und im Flüssigkeitsstrahlwäscher 3 unter einem Druck von mindestens 4 bar mit mehr als 90$^{\circ}$C heißem, höher als 300 Gew.-ppm katalysatorhaltigem und über die Pumpe 23 auf einen Treibstrahldruck von mindestens 5 bar gebrachtem, im wesentlichen aus EDC bestehendem Kreislaufstrom 21 derart in Kontakt gebracht, daß die vorher erläuterten Forderungen für die Chlorabsorption (Stoffaustausch bei einer spezifischen Austauschfläche von mindestens $10^3$ m$^2$ m$^3$ und einer mittleren Verweilzeit zwischen der Chlorzugabe in den Wäscher 3 und dem Reaktionsbeginn mit Ethylen im Static-Mischer 8 von weniger als 120 Sekunden) erfüllt werden. Insbesondere können bei hohem Inertgas-Anteil im Elektrolysechlor über die Leitung 3a im oberen Teil des Wäschers 3 die hierbei im EDC bzw.im Kreislaufstrom nicht gelösten Inertgase abgeführt werden. Der EDC-Kreislaufstrom 4 mit weniger als 3 Gew.-% Chlor wird über die Pumpe 5 auf einen Druck von etwa 2,5 bar über dem jeweiligen Reaktordruck von mehr als 6 bar (bevorzugter Reaktordruck 7 bis 20 bar) gebracht, damit eine Energiedissipationsdichte von mindestens 0,05 kW/m$^3$ im Static-Mischer 8, im Impulsaustauschrohr 9 und zur Einstellung der Umlaufströmung 10 im Reaktor 7 erreicht wird. Mit

einem vergleichbar hohen Vordruck wie beim Strom 4 am Reaktoreintritt wird eine zum Chlor nahezu äquivalente Masse Ethylen über den Static-Mischer 8 in den Reaktor 7 derart eingeleitet, daß sich am Reaktoraustritt eine geringe Chlorkonzentration von 100 bis etwa 300 Gew.-ppm Chlor im EDC-Kreislauf 11 einstellt. Es hat sich als besonders vorteilhaft erwiesen, die als Inhibitor wirkende Luft vor Eintritt der Reaktanden Chlor und Ethylen in den Static-Mischer 8 und der hierbei auftretenden Homogenisierung der Komponenten in den Kreislaufstrom 4 einzuleiten. Die Reaktion findet bei einer Temperatur von 90 bis 200$^\circ$C, vorzugsweise 120 bis 160$^\circ$C, einem Druck von 7 bis 20 bar und einer Energiedissipationsdichte von 0,05 bis 1000 kW/m$^3$ statt. Durch entsprechende Einbauten im Reaktor und geeignete Wahl des Eintritts in den und des Austritts aus dem Reaktor wird eine Fluiddynamik im Reaktor eingestellt, die eine Phasentrennung verhindert. Mit Hilfe des Innenrohrs 9 und der Umlenkung 24 wird die Zirkulationsströmung 10 erzeugt. Das nahezu vollständig abreagierte Reaktionsgemisch verläßt nach einer mittleren Verweilzeit des Ethylens vom Eintritt in den Reaktor 7 bis zur Phasentrennung im Entspannungsgefäß 12 von weniger als 60 Minuten, vorzugsweise von 2 bis 60 Minuten, als Gas-Flüssig-Dispersion 11 den Reaktor 7 und wird in das Entspannungsgefäß 12 eingeleitet. Hier findet eine Phasentrennung in eine gasförmige Phase und eine flüssige Phase statt. Die gasförmige Phase besteht aus Inertgasanteilen der Rohstoffe und der Inhibitoren, nicht umgesetzten Reaktanden Chlor und Ethylen, leichtflüchtigen Reaktionsprodukten und EDC-Dämpfen, die im Gleichgewicht mit den vorher genannten Stoffen unter den hier vorliegenden Prozeßbedingungen stehen. Die flüssige Phase besteht aus katalysatorhaltigem EDC mit geringen Anteilen an gelösten Reaktanden. Die Chlorkonzentration im Kreislaufstrom 11 beträgt am Austritt aus dem Reaktor 7 vorzugsweise 100 bis 200 Gew.-ppm. Über das Entspannungs-

- 12 -

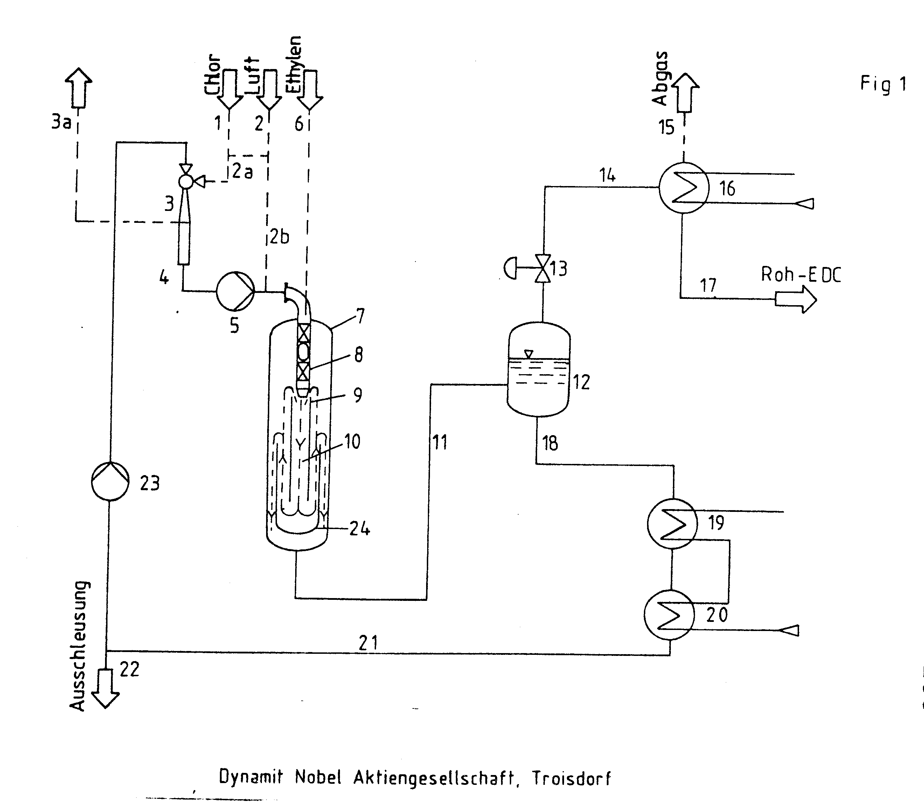

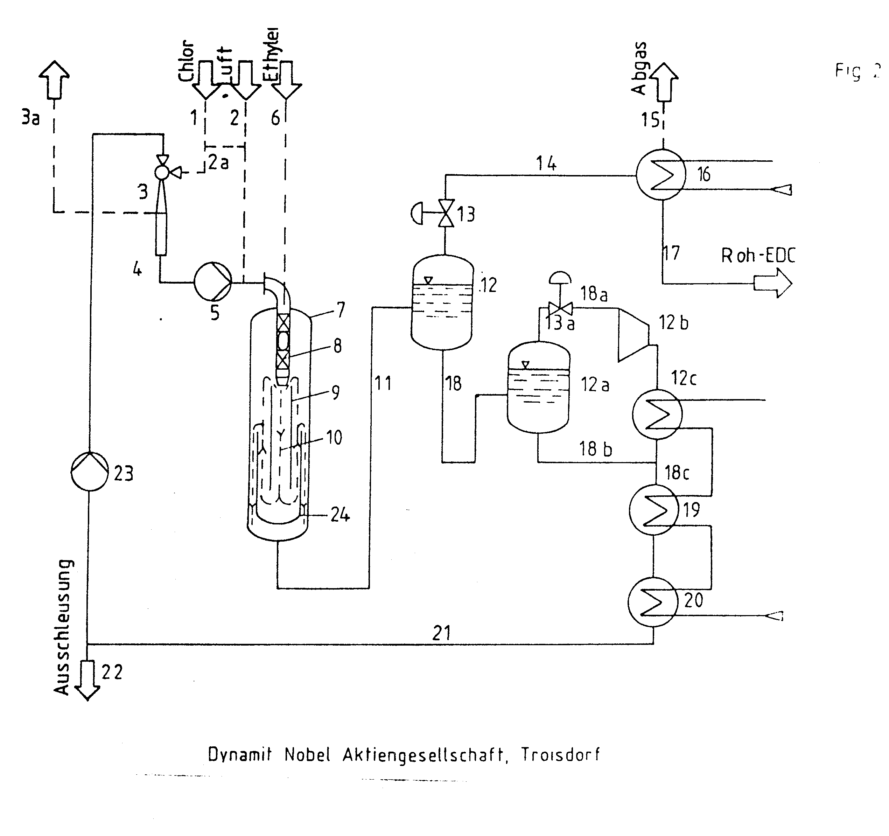

ventil 13 wird der Druck in dem Entspannungsgefäß soweit herabgesetzt, daß unter den vorher genannten Bedingungen gerade soviel EDC verdampft wie im Reaktor 7 gebildet wird. Die Auftrennung des gasförmigen EDC aus dem Gasstrom 14 in den Abgasstrom 15 und den Roh-EDC-Strom 17 erfolgt z.B. durch einfache oder mehrfache direkte oder indirekte Kondensation. In den Figuren 1 und 2 erfolgt die Kondensation beispielsweise in der einfachen indirekten Form im Wärmeaustauscher 16. Neben den Gesichtspunkten der Energierückgewinnung sind es Aspekte der Abgasbehandlung und der Aufarbeitung des Roh-EDC, die für eine optimale Ausgestaltung der Trennung des Roh-EDC aus dem Gasstrom 14 von Bedeutung sind. Der durch die EDC-Verdampfung im Entspannungsgefäß 12 nur geringfügig abgekühlte flüssige EDC-Kreislaufstrom 18 wird einer Energierückgewinnungsstufe, z.B. einer Dampfgewinnungseinrichtung oder der Sumpfheizung einer Destillation, zugeführt. Diese Verfahrensstufe kann z.B. aus mehreren geeigneten Wärmeaustauschern 19 und 20 (s. Fig. 1) bestehen. In einer besonders vorteilhaften Ausgestaltung des Verfahrens wird ein Teil bis zu einem Vielfachen des produzierten EDC aus dem Kreislaufstrom 18 in einem weiteren Entspannungsgefäß 12a (s. Fig. 2) verdampft, in einem Kompressor 12b durch Verdichtung auf eine höhere Temperatur gebracht, anschließend im Wärmeaustauscher 12c, z.B. durch Erzeugung von Wasserdampf, gekühlt und kondensiert.

Im Wärmeaustauscher 12c entsteht dabei höhergespannter Dampf als der Austrittstemperatur des EDC-Kreislaufstromes 11 aus dem Reaktor 7 entspricht. EDC-Kreislaufstrom 18c wird durch die Wärmeaustauscher 19 und 20 vorzugsweise auf eine solche Temperatur gebracht, daß im Wäscher 3 bis zu 3 Gew.-% Chlor absorbiert werden.

- 13 -

Eine diskontinuierliche Ausschleusung einer Teilmenge 22 aus dem Kreislaufstrom 21 ist immer dann erforderlich, wenn der Gehalt an 1.1.2-Trichlorethan und anderen höhersiedenden Chlorkohlenwasserstoffen gleich oder größer als 1 Gew.-% ist und eine Katalysatorkonzentration von 2000 Gew.-ppm Eisen-III-chlorid überschritten wird.

Beispiel 1 (Fig. 1)

Die in Fig. 1 schematisch dargestellte Anlage wurde mit 2200 kg Reinst-Ethylendichlorid (EDC) befüllt. In dieser Kreislaufmasse waren bereits 1000 mg Eisen-III-chlorid pro kg EDC als Katalysator gelöst. Der pH-Wert der Katalysator-Lösung betrug 2,4.

Zur Chlorabsorption im Flüssigkeits-Strahl-Wäscher 3 wurden in die Saugleitung des Wäschers 400 kg/h Elektrolysechlor 1 mit einem Inert-Gas-Anteil von 5 Vol.-% und 2 kg/h Luft 2 über die Leitung 2a gemeinsam eingeleitet und dort in einem Kreislaufstrom von 22 t/h EDC bei einer Temperatur von 99°C und einem Druck im Wäscher von 5 bar innerhalb von etwa 50 Sekunden gelöst. Dabei diente der Kreislaufstrom als sogenannter Treibstrahl und mußte zu diesem Zweck mit der Pumpe 23 auf einen Vordruck von 7,5 bar gebracht werden. Der chlorhaltige Kreislaufstrom 4 wurde mit der Pumpe 5 auf einen Druck von 10 bar gebracht und vor dem Eintritt in den Reaktor 7 über die Leitung 2b mit 1 kg/ h Luft versetzt. Zu diesem chlorhaltigen Kreislaufstrom wurden etwa 153 kg/h Ethylen mit einem Vordruck von 11 bar über den Static-Mischer 8 in den vollständig mit EDC gefüllten Reaktor 7 eingeleitet. Über eine Feinregelung des Ethylenstromes 6 wurde die Chlorkonzentration im EDC-Kreislaufstrom 11 auf etwa 200 Gew.-ppm eingestellt. Die im Reaktor in einer Gas-Flüssig-

- 14 -

Dispersion ablaufende Chlorierungsreaktion von Ethylen zu EDC führte bei einem Reaktorvolumen von 1,1 m$^3$, einer Energiedissipationsdichte im Reaktor von etwa 1 $\frac{kW}{m^3}$ und einem Reaktionsdruck von 8 bar zu einer Temperatur am Austritt des Reaktors von 132$^o$C.

Mit dieser Temperatur tritt der weitestgehend abreagierte Kreislaufstrom 11 in das Entspannungsgefäß 12. Mit einer Standregelung wird über das Entspannungsventil 13 mit den gasförmigen Bestandteilen soviel EDC verdampft, daß sich ein konstanter Stand im Entspannungsgefäß einstellt. Die bei der Trennung des Roh-EDC 17 vom Abgas 15 durch Kondensation im Wärmeaustauscher 16 freigesetzte Wärmemenge wurde beispielsweise zur Kesselspeisewasservorwärmung herangezogen. Der etwa auf 128$^o$C abgekühlte Kreislaufstrom 18 wird in den Wärmeaustauschern 19 und 20 auf 99$^o$C abgekühlt. Hierbei wird ein gesättigter Wasserdampf von 118$^o$C und 1,8 bar erzeugt.

Die Auswertung führte zu folgenden Ergebnissen:

| | |
|---|---|
| Umsatz an Ethylen | 99,6% |
| davon zu EDC | 98,9% |
| ergibt eine Ausbeute von | 98,5% |

der Theorie.

Beispiel 2

Im Gegensatz zum Beispiel 1 wurden bei vergleichbarer Versuchsdurchführung die Massenströme der Reaktanden Chlor 1 auf 200 kg/h und Ethylen 6 auf 77 kg/h sowie die entsprechenden vorher genannten Luftströme 2a und 2b um die Hälfte reduziert.

Bei konstanten Reaktionsbedingungen, wie z.B. der Füll-

- 15 -

menge von 2200 kg, der Katalysatorkonzentration von 1000 Gew.-ppm, dem EDC-Kreislaufstrom von 22 t/h EDC und gleichen Drücken wie im Beispiel 1, wurde lediglich die Temperatur im EDC-Kreislaufstrom 21 von vorher 99°C auf 120°C erhöht. Unter diesen Absorptions- und Reaktionsbedingungen stellte sich eine Temperatur am Austritt des Reaktors von 135°C ein.

Nach der Trennung des gebildeten EDC im Entspannungsgefäß 12 durch Verdampfung stellte sich eine EDC-Kreislauftemperatur im Kreislaufstrom 18 von 133°C ein. Der in den Wärmeaustauschern 19 und 20 auf 120°C abgekühlte Kreislaufstrom 21 wurde zur Herstellung von gesättigtem Wasserdampf mit einer Temperatur von 122°C und einem Druck von ca. 2 bar verwandt.

Die Auswertung führte zu folgenden Ergebnissen:

| | |
|---|---|
| Umsatz an Ethylen | 99,9% |
| davon zu EDC | 99,3% |
| ergibt eine Ausbeute von | 99,2% |

der Theorie.

Beispiel 3 (Figur 2)

Der unter identischen Bedingungen wie im Beispiel 2 durchgeführte Versuch des Beispiels 3 unterscheidet sich darin, daß etwa 1,5 t/h EDC 18a aus dem EDC-Kreislaufstrom 18 von 22 t/h über das Entspannungsventil 13a im Entspannungsgefäß 12a verdampft und dem Dampfkompressor 12b zugeführt wurden. Der EDC-Dampf wurde auf einen Druck von 5,8 bar komprimiert und verließ mit einer Temperatur von etwa 150°C den Verdichter, wurde dann im Wärmeaustauscher 12c kondensiert und mit dem katalysatorhalti-

- 16 -

gen Strom 18b vereinigt. Dieser Kreislaufstrom 18c passierte, wie schon vorher beschrieben, die Wärmeaustauscher 19 und 20. Der hierbei erzeugte gesättigte Wasserdampf hatte im Gegensatz zum Beispiel 2 einen um etwa 1 bar höheren Druck von 3 bar.

Der Ethylenumsatz und die EDC-Ausbeute wurden von dieser Maßnahme nicht beeinflußt und entsprachen den in Beispiel 2 aufgeführten Werten.

### Beispiel 4

Im Gegensatz zum Beispiel 2 wurde bei vergleichbarer Versuchsdurchführung der Kreislaufstrom 21 ebenfalls um die Hälfte reduziert, um bei mit Beispiel 1 vergleichbaren Chlorkonzentrationen die mittlere Verweilzeit bei der Chlorabsorption undim Reaktor entsprechend zu erhöhen. Bei Berücksichtigung einer dementsprechenden Reduzierung der Energiedissipationsdichte auf etwa die Hälfte des in den Beispielen 1 bis 3 ausgewählten Wertes, bei ähnlichen Temperaturen bei der Chlorabsorption im Wäscher 3 und am Austritt des Reaktors wie im Beispiel 1, führte die Auswertung zu folgenden Ergebnissen:

|  |  |
|---|---|
| Umsatz an Ethylen | 99,4% |
| davon zu EDC | 98,0% |
| ergibt eine Ausbeute von | 97,4% |

der Theorie.

Troisdorf, den 16.11.1984
OZ: 84065 (4358)    Me/P.

1  Patentansprüche:

1.
Verfahren zur Herstellung von 1,2-Dichlorethan in einem Reaktor durch Absorption von Chlor in einem heißen, unter erhöhtem Druck stehenden, katalysatorhaltigen, flüssigen Kreislaufstrom, welcher aus chlorierten Kohlenwasserstoffen besteht, und Umsetzung von gasförmigem Ethylen mit dem in der flüssigen Phase gelösten Chlor, g e k e n n - z e i c h n e t  durch folgende Maßnahmen:

a)
die gesamte zur Umsetzung mit dem gasförmigen Ethylen erforderliche Chlormenge wird außerhalb des Reaktors (7) im Kreislaufstrom (21) absorbiert und in Form einer Lösung in den Reaktor eingeleitet,

b)
die Absorption des Chlors im Kreislaufstrom erfolgt bei einer Temperatur oberhalb 90°C, einem Druck von mehr als 4 bar und mit einer mittleren Verweilzeit von weniger als 120 Sekunden,

c)
gasförmiges Ethylen wird in den Reaktor eingeleitet,

d)
aus dem gasförmigen Ethylen und dem chlorhaltigen, flüssigen Kreislaufstrom wird im Reaktor mittels einer Mischzone und Strömungseinbauten eine Dispersion erzeugt, an deren Phasengrenzfläche bei einer Energiedissipationsdichte von 0,05 bis 1000 kW/m$^3$, einer Temperatur von 90 bis 200°C und einem Druck von 7 bis 20 bar die Reaktion zwischen Ethylen und Chlor abläuft,

- 2 -

e)

die nichtumgesetzten gasförmigen Bestandteile verlassen den Reaktor im Kreislaufstrom dispergiert,

f) die Chlorkonzentration im Kreislaufstrom (11) aus dem Reaktor (7) beträgt mindestens 100 Gew.-ppm,

g) der Katalysator besteht aus einem Metallchlorid oder einer ein Metallchlorid enthaltenden Verbindung.

2.

Verfahren nach Anspruch 1, dadurch gekennzeichnet, daß die Reaktion zwischen Ethylen und Chlor bei einer Temperatur von 120 bis 160°C stattfindet.

3.

Verfahren nach Anspruch 1 oder 2, dadurch gekennzeichnet, daß als Katalysator Eisen-III-chlorid oder eine Eisen-III-chlorid enthaltende Verbindung in einer Menge von 30 bis 3000 Gew.-ppm, berechnet als $FeCl_3$, im Kreislaufstrom (21) enthalten ist.

4.

Verfahren nach einem der Ansprüche 1 bis 3, dadurch gekennzeichnet, daß bei der Reaktion zwischen Ethylen und Chlor zusätzlich ein Inhibitor oder mehrere Inhibitoren zur Verhinderung der Nebenproduktbildung anwesend ist bzw. sind.

5.

Verfahren nach Anspruch 4, dadurch gekennzeichnet, daß als Inhibitor Sauerstoff oder ein Sauerstoff enthaltendes

- 3 -

Gas in einem Anteil von 0,05 - 0,3 Gew.-%, berechnet als $O_2$ und bezogen auf die zur Reaktion eingesetzte Chlormenge, verwendet wird.

6.
Verfahren nach Anspruch 4 oder 5, dadurch gekennzeichnet, daß die Inhibitoren in die Mischzone des Reaktors (7) eingeleitet und dort mit einer Energiedissipationsdichte von 10 bis 1000 $kW/m^3$ verteilt werden.

7.
Verfahren nach einem der Ansprüche 1 bis 6, dadurch gekennzeichnet, daß die im Kreislaufstrom (21) nicht löslichen Bestandteile des eingesetzten Chlorgases sowie der Inhibitoren vor dem Eintritt in die Reaktionszone des Reaktors (7) entfernt werden.

8.
Verfahren nach einem der Ansprüche 1 bis 7, dadurch gekennzeichnet, daß die mittlere Verweilzeit des im Kreislaufstrom dispers verteilten Ethylens vom Eintritt in den Reaktor (7) bis zur Phasentrennung im Entspannungsgefäß (12) weniger als 60 Minuten beträgt.

9.
Verfahren nach einem der Ansprüche 1 bis 8, dadurch gekennzeichnet, daß die Chlorkonzentration im Kreislaufstrom (11) am Austritt aus dem Reaktor (7) 100 bis 200 Gew.-ppm beträgt.

10.
Verfahren nach einem der Ansprüche 1 bis 9, dadurch gekennzeichnet, daß die Trennung der nicht umgesetzten Mengen an Ethylen und Chlor, der nicht gelösten Bestandteile der Inhibitoren und des eingesetzten Chlorgases

- 4 -

sowie der leichtflüchtigen Reaktionsprodukte vom produkthaltigen Kreislaufstrom (21) im Entspannungsgefäß (12)
erfolgt.

11.
Verfahren nach einem der Ansprüche 1 bis 10, dadurch gekennzeichnet, daß ein Teil bis zu einem Vielfachen des
produzierten EDC aus dem Kreislaufstrom (18) in einem
weiteren Entspannungsgefäß (12a) verdampft, anschließend
durch Verdichtung auf eine höhere Temperatur gebracht und,
z.B. durch Erzeugung von Wasserdampf, gekühlt und kondensiert wird.

Fig 1

Fig 2

Dynamit Nobel Aktiengesellschaft, Troisdorf

0187206

Europäisches
Patentamt

**0187206**

Nummer der Anmeldung

**EUROPÄISCHER RECHERCHENBERICHT**

EP 85 11 2964

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,Y | DE-C-3 340 624  (DYNAMIT NOBEL)<br>* Ansprüche;  Spalte  4, Absätze 2,6 * | 1-5 | C 07 C  19/045<br>C 07 C  17/02 |
| | --- | | |
| D,X | EP-A-0 075 742  (HOECHST)<br>* Ansprüche; Seiten 6-7 * | 1-5 | |
| | ----- | | |

|  |  |
|---|---|
| | **RECHERCHIERTE SACHGEBIETE** (Int Cl 4) |
| | C 07 C  17/00<br>C 07 C  19/00 |

Der vorliegende Recherchenbericht wurde für alle Patentansprüche erstellt.

| Recherchenort | Abschlußdatum der Recherche | Prüfer |
|---|---|---|
| DEN HAAG | 29-04-1986 | VAN GEYT J.J.A. |